# EUROPEAN PATENT SPECIFICATION

(11) **EP 2 651 883 B1**
(45) Date of publication and mention of the grant of the patent: **25.02.2015**
(21) Application number: 11793456.2
(22) Date of filing: 09.12.2011
(51) Int. Cl.: C07C 409/32, C08F 4/34, C07C 409/34

(54) **PROCESS FOR PREPARING AN ORGANIC SOLUTION OF A DIALKYL PEROXYDICARBONATE**
VERFAHREN ZUR HERSTELLUNG EINER ORGANISCHEN LÖSUNG EINES DIALKYLPEROXYDICARBONATS
PROCÉDÉ POUR LA PRÉPARATION D'UNE SOLUTION ORGANIQUE D'UN PEROXYDICARBONATE DE DIALKYLE

(30) Priority: 14.12.2010 FR 1060471
(43) Date of publication of application: 23.10.2013
(73) Proprietor: SOLVAY SA, 1120 Bruxelles (BE)
(72) Inventor: BODART, Vincent, B-5001 Namur (BE)
(74) Representative: Vande Gucht, Anne
(86) International application number: PCT/EP2011/072331
(87) International publication number: WO 2012/080124

(56) References cited:
- EP-A1- 0 893 438
- WO-A1-01/32613
- WO-A1-97/27229
- WO-A1-2004/096762
- FR-A5- 2 161 239
- GB-A- 915 009
- US-A- 5 541 151
- MUELLER P A ET AL: "Modeling of vinylidene fluoride heterogeneous polymerization in supercritical carbon dioxide", MACROMOLECULES 20050809 AMERICAN CHEMICAL SOCIETY US, vol. 38, no. 16, 9 August 2005 (2005-08-09), pages 7150-7163, XP002633407, DOI: DOI:10.1021/MA0504522
- STRAIN F ET AL: "Esters of peroxycarbonic acids", JOURNAL OF THE AMERICAN CHEMICAL SOCIETY, AMERICAN CHEMICAL SOCIETY, US, vol. 72, 1 January 1950 (1950-01-01), pages 1254-1263, XP002163481, ISSN: 0002-7863, DOI: DOI:10.1021/JA01159A052

## Description

The present invention relates to organic dialkyl peroxydicarbonate solutions and also to a process for the preparation thereof. It also relates to a process for preparing polymers derived from halogenated ethylenically unsaturated monomers using these solutions.

Dialkyl peroxydicarbonates constitute initiators that are valued for initiating the aqueous suspension polymerization of halogenated ethylenically unsaturated monomers, and more particularly of ethylenically unsaturated monomers containing chlorine, such as vinyl chloride. Dialkyl peroxydicarbonates containing short alkyl chains, such as diethyl, diisopropyl and dibutyl peroxydicarbonates, constitute initiators that are particularly valued due to their high activity at the standard temperatures for the polymerization of vinyl chloride. However, they have the drawback of being unstable, so that their storage in the pure state presents very serious risks.

Although it is possible to prepare them directly in the polymerization reactor ("*in situ*"), this process is not particularly beneficial from the point of view of the productivity since it is necessary to precede each polymerization cycle with the "*in situ"* synthesis of the initiator. This *"in situ*" process for preparing the initiator also does not allow the delayed feeding of the reactor, i.e. feeding the reactor during the polymerization.

To overcome this drawback it has already been proposed to prepare, via reaction of an alkyl haloformate with a peroxide compound in the presence of water and a solvent that is immiscible with water (preferably having a boiling point below 100°C at atmospheric pressure if it is desired to eliminate it before the polymerization, such as halogenated hydrocarbons, pentane, hexane or cyclohexane), just the right amount of dialkyl peroxydicarbonate, outside of the polymerization reactor (technique commonly referred to as *"ex situ*") and immediately before the polymerization. The initiator solution thus obtained is then introduced *in toto* (organic phase and aqueous phase) into the polymerization reactor which is then charged for the purpose of the polymerization (document GB-A-1484675). This process necessitates always producing just the right amount of initiator immediately before the polymerization. This process does not make it possible, furthermore, to have a dialkyl peroxydicarbonate solution that can be stored in complete safety and that can be used at any moment, especially for delayed feeding. Moreover, either the solvent present in the dialkyl peroxydicarbonate solution must be eliminated before the polymerization or, if it is not, it may lead to polymers that contain undesirable organic impurities.

In order to be able to have an organic dialkyl peroxydicarbonate solution that can be stored in complete safety and that can be used at any moment, it has already been proposed to use agents known as "phlegmatizers", i.e. agents that have a stabilizing action on the tendency of the dialkyl peroxydicarbonate towards exothermic decomposition. Examples of such phlegmatizing agents have been mentioned in document WO 2004/000799-A1 (D1), page 1, lines 11 to 25. They may be, *inter alia,* phenol, hydroquinone, nitromethane, cyclohexene, organic hydroperoxides, oximes and cyclic alpha-diketones.

To attempt to solve the same problem, it has also been proposed to prepare said solution by reacting, in an aqueous medium, an alkyl haloformate with the required amount of an inorganic peroxide, and by then separating the dialkyl peroxydicarbonate obtained by extraction with a water-insoluble organic solvent. Commonly used water-insoluble organic solvents are hydrocarbons and esters. As hydrocarbons, xylene, perchloroethylene, perhalogenated saturated hydrocarbons (document BE 638304 A1) and isododecane (document WO 2007/042313 A2) have especially been described. As esters that can be used, dialkyl alkanedicarboxylates that are liquid and are insoluble in water, and more particularly adipates (document US 2001/0031846 A1) have especially been described.

These phlegmatizing agents and these organic solvents have drawbacks. It is necessary to use them in relatively large amounts because, on the one hand, they may not be very good solvents of the dialkyl peroxydicarbonates and, on the other hand, it is necessary to greatly dilute the organic dialkyl peroxydicarbonate solution to ensure the stability thereof. These compounds are hence found in the form of undesirable organic impurities in the halogenated polymers prepared using said solution and with which they are not very compatible. Furthermore, in the preferred case where the halogenated polymer prepared is a polyvinyl chloride (PVC) and when the organic solvent is an adipate, the latter has a plasticizing action on the PVC, which is undesirable should it be desired to manufacture a rigid PVC.

It has already been attempted to overcome these drawbacks by proposing (see document (D1) mentioned above) to use phlegmatizing agents described as having favourable effects, both on the stability of the peroxide composition in which they are incorporated and on the preparation of the polymer (for example PVC) carried out in the presence of this composition. Among these effects is the "reactive" nature of the phlegmatizing agent which results in its "consumption" during the polymerization reaction with, as the main consequence, a reduction in the amount of unbound phlegmatizing agent and a further reduced content of volatile matter in the polymer obtained.

"Reactive" phlegmatizing agents that are preferred according to this document are C₄-C₁₂ alpha-olefins, cyclic alkenes and mixtures thereof. The degree of reactivity of these agents is such that, preferably, less than 25% of their weight can be extracted from the polymer obtained.

Document US 4131728 A (D2) itself describes the polymerization of ethylenically unsaturated monomers in the presence of organic peroxides being in the form of an intimate mixture of a shock-sensitive organic peroxide and an ethylenically unsaturated monomer that does not homopolymerize. As monomers that correspond to this definition, mention is especially made of maleates and fumarates.

The "reactive" phlegmatizing agents and the unsaturated monomers proposed respectively in documents (D1) and (D2) do not however satisfactorily solve the problem of the presence of harmful volatile matter in the polymer obtained. Indeed, the incorporation, into the polymer synthesized in the presence of peroxide-containing initiators in the composition in which they come, of these phlegmatizing agents and unsaturated monomers is incomplete and does not make it possible to avoid a treatment of the polymer in order to eliminate them therefrom.

WO2004/096762 discloses an organic solution of dialkyl peroxydicarbonate comprizing methyl crotonate, and the use of such stabilized composition in a process of polymerization of vinyl chloride.

US 5541151 discloses a process for the preparation of an organic solution of dialkyl peroxydicarbonate, in which an alkyl haloformate is reacted with aqueous hydrogen peroxide in the presence of a solvent containing a carbon-carbon double bond and either a nitrile or an alkyne group, and the use of such stabilized composition in a process of polymerization of vinyl chloride.

The present invention aims to provide an organic solution of a dialkyl peroxydicarbonate which does not have these drawbacks, owing to the use of solvents that are incorporated significantly better into the macromolecular chains of the halogenated polymers prepared using said solution, making it particularly suitable for the preparation of polymers that are virtually free of undesirable organic impurities, and more particularly chlorine-containing polymers (including PVC), via aqueous suspension polymerization.

The present invention therefore relates mainly to a process for preparing an organic solution of a dialkyl peroxydicarbonate in a liquid organic solvent (S), carried out at least partly in the presence of the liquid organic solvent (S) and comprising at least one step during which, in a medium containing water, an alkyl haloformate is reacted with an inorganic peroxide, the liquid organic solvent (S) being chosen from the compounds corresponding to the empirical formula:

A - (CₙH(₂ₙ₋₂₎) - B (I)

in which:
- A represents a halogen functional group (A1) or a functional group (A2) corresponding to the structure R-C(=O)-O- in which R is chosen from hydrogen; chlorine; substituted or unsubstituted, linear or branched C₁-C₂₀ alkyl radicals; substituted or unsubstituted C₃-C₂₂ alkenyl radicals; substituted or unsubstituted C₆-C₁₀ cycloalkyl radicals; substituted or unsubstituted C₇-C₁₂ aralkyl radicals, and substituted or unsubstituted C₆-C₁₀ aryl radicals;
- B represents either a hydrogen atom or, when A is not (A2), a second halogen functional group (A1); and
- n is such that 3 ≤ n ≤ 8.

The expression "organic solution of a dialkyl peroxydicarbonate in a liquid organic solvent (S)" is understood to mean, for the purposes of the present description, any solution comprising a dialkyl peroxydicarbonate and a liquid organic solvent (S). The expression "comprising" is understood to mean, for the purposes of the present description, that this solution may comprise, besides the dialkyl peroxydicarbonate and the liquid organic solvent (S), other constituents, such as in particular water (for example resulting from the preparation of the organic solution).

According to a first embodiment of the present invention, the organic solution according to the invention comprises the dialkyl peroxydicarbonate, the liquid organic solvent (S) and water, in particular water resulting from the preparation of the organic solution, i.e. water which may especially contain water-soluble compounds resulting from this preparation.

According to a second embodiment of the present invention which is preferred, the organic solution according to the invention is constituted essentially of the dialkyl peroxydicarbonate and the liquid organic solvent (S).

The expression "constituted essentially" is understood to mean, for the purposes of the present description, that the organic solution is water-free. The term "free" is understood to mean an organic solution comprising less than 5% by weight of water, preferably less than 1% by weight of water.

Irrespective of the embodiment, the final dialkyl peroxydicarbonate concentration of the organic solution is advantageously greater than 5% by weight, preferably greater than 10% by weight, particularly preferably greater than 15% by weight of the total weight of dialkyl peroxydicarbonate plus liquid organic solvent (S).

Irrespective of the embodiment, the final dialkyl peroxydicarbonate concentration of the organic solution is advantageously less than 90% by weight, preferably less than 80% by weight, particularly preferably less than 75% by weight and very particularly preferably less than 50% by weight of the total weight of dialkyl peroxydicarbonate plus liquid organic solvent (S).

Organic solutions for which the final dialkyl peroxydicarbonate concentration is between 25% and 40% by weight of the total weight of dialkyl peroxydicarbonate plus liquid organic solvent (S) are particularly advantageous, especially in view of their subsequent use.

Although the expression "organic solution of a dialkyl peroxydicarbonate" is used in the singular in the present description, this does not mean that an organic solution containing several different dialkyl peroxydicarbonates is excluded from the scope of the invention. Preferably, the organic solution of a dialkyl peroxydicarbonate in a liquid organic solvent (S) according to the invention comprises a single dialkyl peroxydicarbonate.

The dialkyl peroxydicarbonate is most generally a dialkyl peroxydicarbonate having linear or branched alkyl radicals that contain from 2 to 9 carbon atoms. Preferably, the dialkyl peroxydicarbonate is a dialkyl peroxydicarbonate having alkyl radicals that contain from 2 to 4 carbon atoms (i.e. ethyl, propyl or isopropyl, and butyl or isobutyl radicals). Particularly preferably, the dialkyl peroxydicarbonate is a dialkyl peroxydicarbonate having alkyl radicals that contain 2 or 3 carbon atoms (i.e. ethyl or isopropyl radicals). One dialkyl peroxydicarbonate that is very particularly preferred is diethyl peroxydicarbonate.

The alkyl haloformate used in the process according to the invention is usually and advantageously an alkyl chloroformate, the alkyl radical of which corresponds to the definitions given above in connection with the dialkyl peroxydicarbonate.

The inorganic peroxide used in the process according to the invention is usually calcium or sodium peroxide or else aqueous hydrogen peroxide solution. In the latter case, it is advisable to additionally introduce, into the medium containing water, a base, such as calcium hydroxide or else sodium hydroxide. Preferably, the inorganic peroxide is aqueous hydrogen peroxide solution and sodium hydroxide is then added to the reaction medium.

In the remainder of the present description, the expression "inorganic peroxide" is understood to mean both calcium or sodium peroxide alone, and aqueous hydrogen peroxide solution to which calcium or sodium hydroxide has been added.

The amount of inorganic peroxide is customarily less than or equal to the stoichiometric amount. In the preferred case of the use of aqueous hydrogen peroxide solution and sodium hydroxide as inorganic peroxide, the deficit in the amount thereof, relative to stoichiometry, is generally less than or equal to 5 mol% relative to the amount of alkyl haloformate. The deficit in the amount relative to stoichiometry does not necessarily have to be the same for the aqueous hydrogen peroxide solution and the sodium hydroxide. A difference of 3% for the sodium hydroxide and of 4% for the aqueous hydrogen peroxide solution, relative to the amount of alkyl haloformate, customarily gives good results.

The nature of the liquid organic solvent (S) (also known more simply as "solvent (S)" in the present description) is an important characteristic of the process according to the invention. Advantageously, this solvent (S) is not very soluble in water. The expression "solvent not very soluble in water" is understood in the present description to mean a solvent having a solubility in water at room temperature (around 20°C) and under atmospheric pressure that is advantageously less than 10 g/l and more particularly less than 5 g/l.

Although the expression "organic solution in a liquid organic solvent (S)" is used in the singular in the present description, this does not mean that an organic solution containing several different solvents of formula (I) is excluded from the scope of the invention. Preferably, the organic solution of a dialkyl peroxydicarbonate in a liquid organic solvent (S) according to the invention comprises a single liquid organic solvent (S).

The expression "liquid organic solvent" is understood to mean solvents that are liquids under the operating conditions - defined further on - prevailing during the formation of the dialkyl peroxydicarbonate according to the process of the invention.

Advantageously, this solvent (S) has the property of being incorporated into the growing macromolecular chains of the polymers, derived from halogenated ethylenically unsaturated monomers, synthesized in the presence of the organic solution of a dialkyl peroxydicarbonate prepared according to the invention.

This solvent (S) is chosen from the compounds corresponding to the empirical formula:

A - (CₙH(₂ₙ₋₂₎) - B (I)

in which:
- A represents a halogen functional group (A1) or a functional group (A2) corresponding to the structure R-C(=O)-O- in which R is chosen from hydrogen; chlorine; substituted or unsubstituted, linear or branched C₁-C₂₀ alkyl radicals; substituted or unsubstituted C₃-C₂₂ alkenyl radicals; substituted or unsubstituted C₆-C₁₀ cycloalkyl radicals; substituted or unsubstituted C₇-C₁₂ aralkyl radicals, and substituted or unsubstituted C₆-C₁₀ aryl radicals;
- B represents either a hydrogen atom or, when A is not (A2), a second halogen functional group (A1); and
- n is such that 3 ≤ n ≤ 8.

In the formula (I), the halogenated functional group (A1) is preferably chlorine.

According to a first family of solvents (S), the solvent (S) is advantageously chosen from the compounds corresponding to the formula (I) in which A represents a halogenated functional group (A1) and B a hydrogen atom. These compounds are then preferably chosen from:
- (1) C₃ - C₈ chloro-1-alkenes in which the alkenyl radical is linear or branched; in these compounds (1), the carbon-carbon double bond is therefore terminal;
- (2) trans and cis isomers, and the mixture thereof, of C₃ - C₈ n₁-chloro-n₂-alkenes in which the alkenyl radical is linear or branched, n₁ being between 1 and the number of carbon atoms of the chloroalkene and representing the position of the chlorine atom and n₂ being greater than 1 and less than the number of carbon atoms of the chloroalkene minus 1 and representing the position of the carbon-carbon double bond; in these compounds (2), the carbon-carbon double bond is not therefore terminal; among these isomers, the more stable trans isomers are preferred.

Examples of compounds (1) that can be used as solvents (S) are:
- 1-, 2- or 3-chloro-1-propenes;
- 1-, 2-, 3- or 4-chloro-1-butenes; 3-chloro-2-methyl-1-propene;
- 1-, 2- or 4-chloro-1-pentenes;
- 6-chloro-1-hexene;
- 3- or 7-chloro-1-heptenes;
- 3- or 8-chloro-1-octenes.

Examples of compounds (2) that can be used as solvents (S) are the isomers:
- 1- or 2-chloro-2-butenes;
- 2- or 5-chloro-2-pentenes; 1-chloro-3-pentene;
- 1- or 3-chloro-3-hexenes;
- 1-chloro-2-heptene;
- 1-chloro-2-octene and 8-chloro-3-octene.

Among the compounds of this first family, the solvent (S) is particularly preferably chosen from C₃ - C₈ chloro-1-alkenes in which the alkenyl radical is linear or branched. The solvent (S) is very particularly preferably chosen from the C₃ - C₄ chloro-1-alkenes in which the alkenyl radical is linear or branched. The solvent (S) is very particularly preferably indeed chosen from 3-chloro-1-propene (allyl chloride) and 3-chloro-2-methyl-1-propene.

According to a second family of solvents (S), the solvent (S) is advantageously chosen from the compounds corresponding to the formula (I) in which A represents a functional group (A2) and B a hydrogen atom. The functional group (A2) corresponds to the structure R-C(=O)-O- in which R is chosen from hydrogen, chlorine and substituted or unsubstituted, linear or branched C₁-C₂₀ alkyl radicals; substituted or unsubstituted C₃-C₂₂ alkenyl radicals; substituted or unsubstituted C₆-C₁₀ cycloalkyl radicals; substituted or unsubstituted C₇-C₁₂ aralkyl radicals, and substituted or unsubstituted C₆-C₁₀ aryl radicals. When these radicals are substituted, the substituent is preferably chlorine.

These compounds are then preferably chosen from:
- allyl (prop-2-enyl), methallyl (2-methylprop-2-enyl), pentenyl, hexenyl, heptenyl and octenyl formates or chloroformates;
- allyl, methallyl, pentenyl, hexenyl, heptenyl and octenyl acetates or chloroacetates;
- allyl, methallyl, pentenyl, hexenyl, heptenyl and octenyl propionates, butyrates, valerates, caproates, heptanoates, caprylates, nonanoates, caprates, laurates, myristates, palmitates or stearates;
- allyl, methallyl, pentenyl, hexenyl, heptenyl and octenyl propenoates, butenoates, myristoleates and palmitatoleates;
- allyl, methallyl, pentenyl, hexenyl, heptenyl and octenyl salicylates and benzoates.

Preferably, R is chosen from hydrogen and linear C₁-C₆ alkyl radicals.

Among the compounds of this second family, the solvent (S) is particularly preferably chosen from allyl formate, acetate, propionate and butyrate. 3-Aceto-1-propene (allyl acetate) is very particularly preferred.

According to a third family of solvents (S), the solvent (S) is advantageously chosen from the compounds corresponding to the formula (I) in which A and B each represent a halogenated functional group (A1). These compounds are then preferably chosen from stereoisomers, and mixtures thereof, of 1,3-dichloropropene, 1,3-dichloro-2-methylpropene, 1,4-dichlorobutenes, 1,5-dichloropentene, 1,6-dichlorohexene, 1,7-dichloroheptenes and 1,8-dichlorooctenes.

Among the compounds of this third family, the solvent (S) is particularly preferably chosen from stereoisomers of 1,3-dichloropropene, and mixtures thereof.

Known operating conditions of the reaction of an alkyl haloformate with an inorganic peroxide, in a medium containing water and at least partly in the presence of the liquid organic solvent (S), may all be able to be used within the context of the process for preparing the organic solution of a dialkyl peroxydicarbonate according to the invention.

The expression "medium containing water" is understood to mean, within the present description, both a medium constituted exclusively of water and a two-phase mixture containing an organic phase constituted essentially of the solvent (S) and an aqueous phase.

The reaction between the alkyl haloformate and the inorganic peroxide is advantageously carried out with vigorous stirring. The temperature of the reaction is usually maintained at a value located between -5°C and +30°C, preferably between 0°C and +15°C. The total duration of the preparation of the dialkyl peroxydicarbonate is advantageously governed by the duration of the addition of the inorganic peroxide to the medium containing the alkyl haloformate. In the preferred case of the use of aqueous hydrogen peroxide solution and sodium hydroxide as inorganic peroxide, the total duration of the preparation of the dialkyl peroxydicarbonate is advantageously governed by the duration of the addition of the sodium hydroxide to the medium containing the alkyl haloformate and the hydrogen peroxide, which customarily varies from a few tens of minutes to a few hours.

According to a first variant (variant 1), the preparation of the organic solution of a dialkyl peroxydicarbonate according to the invention is carried out by introducing, in one step, the alkyl haloformate, the inorganic peroxide and the solvent (S) into a medium containing water, while keeping the reaction mixture thus obtained under vigorous stirring. Conventional dispersants, such as cellulose derivatives and polyvinyl alcohols, may optionally be added to this reaction mixture.

According to this variant 1, the amount of water advantageously represents between 10 and 1000, preferably between 25 and 100 parts by weight of the haloformate used.

According to this variant 1, the amount of solvent (S) advantageously represents between 100 and 0.20, preferably between 10 and 1 parts by weight of the haloformate used.

According to this first variant, the addition of the solvent (S) to the medium containing water may be carried out from the start of the reaction for forming the dialkyl peroxydicarbonate, i.e. at the moment of addition of the inorganic peroxide to the alkyl haloformate, to avoid having to handle pure dialkyl peroxydicarbonate, in view of its explosive nature. It is however possible to add the solvent (S) to the medium containing water and the reactants (alkyl haloformate and inorganic peroxide) at the latest up to 60 minutes, preferably 40 minutes, and more particularly 15 minutes after the end of the introduction of the reactants. In practice, the solvent (S) will advantageously be added from the start of the reaction for preparing the peroxydicarbonate or at the latest around 10 minutes after the end of the introduction of the reactants, in particular of the inorganic peroxide.

According to one particular method of operation of this variant 1 for preparing the organic solution of a dialkyl peroxydicarbonate (variant 1.1), the alkyl haloformate is introduced in the form of a solution into the solvent (S).

In the case of variant 1 for preparing the organic solution according to the invention, the definition "organic solution of a dialkyl peroxydicarbonate in a liquid organic solvent (S)" denotes an organic solution comprising the dialkyl peroxydicarbonate, the liquid organic solvent (S) and water, in particular water resulting from the preparation of the organic solution, i.e. water which may especially contain water-soluble compounds resulting from this preparation.

According to a second variant (variant 2), the preparation of the organic solution of a dialkyl peroxydicarbonate according to the invention is carried out in two steps.

In this case, the dialkyl peroxydicarbonate is advantageously prepared in a first step, by reacting, in a medium containing water and optionally the solvent (S), the alkyl haloformate with the inorganic peroxide (the operating conditions of this first step are advantageously similar to those defined and stated above (variant 1) unless otherwise indicated) and in a second step (the beginning of which advantageously corresponds to stopping the stirring to which the reaction mixture of the first step was subjected), the dialkyl peroxydicarbonate prepared during this first step is separated. This separation may be carried out according to any known and appropriate method for separating two liquid phases, such as settling, centrifugation, etc. In the event that the solvent (S) is still not present, it is advantageously introduced at this stage as extraction means for separating the dialkyl peroxydicarbonate formed from the aqueous phase.

Advantageously, the phases are left to settle after having stopped the stirring and the organic phase is separated from the aqueous phase in order to collect a pure solution of the dialkyl peroxydicarbonate in the solvent (S).

In the case of variant 2, the amount of solvent (S) used for the extraction is not critical provided that it is at least sufficient to save the organic solution of a dialkyl peroxydicarbonate according to the invention from having an explosive nature. It goes without saying that it will depend in particular on the degree of solubility of the dialkyl peroxydicarbonate in the chosen solvent.

In the case of variant 2, which is preferred for the preparation of the organic solution according to the invention, the definition "organic solution of a dialkyl peroxydicarbonate in a liquid organic solvent (S)" denotes an organic solution constituted essentially of the dialkyl peroxydicarbonate and of the solvent (S) as defined above.

In the case of variant 2 of the process for preparing the organic solution of a dialkyl peroxydicarbonate according to the invention, and when the density of the solvent (S) is less than that of the aqueous phase, an inorganic salt may advantageously be used. This inorganic salt is then advantageously introduced-independently of any salt generated by the reaction between the alkyl haloformate and the inorganic peroxide - into the aqueous phase in a sufficient amount to bring its density to a value greater than the density of the organic solution produced in the first step. The density of the aqueous phase is then preferably at least equal to 1.05 and more particularly still at least equal to 1.10.

The nature of the salt used is not particularly critical. In principle, any inorganic salt that does not interfere with the reaction for forming the dialkyl peroxydicarbonate and which does not precipitate under the reaction conditions is suitable. As non-limiting examples of such salts, mention may be made, for example, of alkali metal and alkaline-earth metal halides and in particular chlorides. Preferably, alkali metal chlorides are used. According to one particularly advantageous embodiment, sodium chloride is used.

The fact of preparing the peroxydicarbonate in a densified aqueous medium advantageously improves the efficiency of the separation of the dialkyl peroxydicarbonate in solution.

Another subject of the present invention is an organic solution of a dialkyl peroxydicarbonate in a liquid organic solvent (S) chosen from the compounds corresponding to the empirical formula:

A - (CₙH(₂ₙ₋₂₎) - B (I)

in which:
- A represents a halogen functional group (A1) or a functional group (A2) corresponding to the structure R-C(=O)-O- in which R is chosen from hydrogen; chlorine; substituted or unsubstituted, linear or branched C₁-C₂₀ alkyl radicals; substituted or unsubstituted C₃-C₂₂ alkenyl radicals; substituted or unsubstituted C₆-C₁₀ cycloalkyl radicals; substituted or unsubstituted C₇-C₁₂ aralkyl radicals, and substituted or unsubstituted C₆-C₁₀ aryl radicals;
- B represents either a hydrogen atom or, when A is not (A2), a second halogen functional group (A1); and
- n is such that 3 ≤ n ≤ 8.

All the definitions, preferences and limitations mentioned and described above can be applied, *mutatis mutandis*, to the organic solution according to the invention.

The organic solution of a dialkyl peroxydicarbonate in a liquid organic solvent (S) is preferably prepared by the process for preparing an organic solution of a dialkyl peroxydicarbonate in a liquid organic solvent (S) according to the invention.

The present invention also relates to a process for preparing a polymer derived from one or more halogenated ethylenically unsaturated monomers, comprising a polymerization step during which at least one halogenated ethylenically unsaturated monomer is polymerized in aqueous suspension or in aqueous microsuspension in the presence of an initiator comprising at least one dialkyl peroxydicarbonate, used in the polymerization at least partly in the form of an organic solution in a liquid organic solvent (S) chosen from the compounds corresponding to the empirical formula:

A - (CₙH(₂ₙ₋₂₎) - B (I)

in which:
- A represents a halogen functional group (A1) or a functional group (A2) corresponding to the structure R-C(=O)-O- in which R is chosen from hydrogen; chlorine; substituted or unsubstituted, linear or branched C₁-C₂₀ alkyl radicals; substituted or unsubstituted C₃-C₂₂ alkenyl radicals; substituted or unsubstituted C₆-C₁₀ cycloalkyl radicals; substituted or unsubstituted C₇-C₁₂ aralkyl radicals, and substituted or unsubstituted C₆-C₁₀ aryl radicals;
- B represents either a hydrogen atom or, when A is not (A2), a second halogen functional group (A1); and
- n is such that 3 ≤ n ≤ 8.

In the remainder of the present description, the expressions "halogenated ethylenically unsaturated monomer", "polymer derived from one or more halogenated ethylenically unsaturated monomers" and "dialkyl peroxydicarbonate" are used without distinction in the singular and in the plural.

All the definitions, preferences and limitations mentioned and described above can be applied, *mutatis mutandis*, to the process for preparing a polymer according to the invention.

According to the present invention, the polymers derived from one or more halogenated ethylenically unsaturated monomers may be both the homopolymers of these monomers and the copolymers that these monomers form with themselves and/or with at least one unhalogenated ethylenically unsaturated monomer. In other words, these polymers comprise advantageously at least 50%, preferably at least 60%, particularly preferably at least 70% by weight of monomer units derived from a halogenated ethylenically unsaturated monomer. This halogenated ethylenically unsaturated monomer is preferably chosen from chlorinated ethylenically unsaturated monomers and fluorinated ethylenically unsaturated monomers, very particularly from chlorinated ethylenically unsaturated monomers.

The expression "polymers derived from fluorinated ethylenically unsaturated monomers" is understood to mean the homopolymers of these monomers and the copolymers that these monomers form with at least one other halogenated ethylenically unsaturated monomer and/or one other unhalogenated ethylenically unsaturated monomer such as ethylene, vinyl acetate and acrylic or methacrylic monomers.

The expression "fluorinated ethylenically unsaturated monomers" is understood to mean the ethylenically unsaturated fluorinated monomers that are aliphatic and that contain one or more fluorine atoms. As examples of fluorinated ethylenically unsaturated monomers for which the number of fluorine atoms is equal to 1, mention may be made of allyl fluoride and vinyl fluoride. As an example of a fluorinated ethylenically unsaturated monomer for which the number of fluorine atoms is equal to 2, mention may be made of vinylidene fluoride. As examples of other fluorinated ethylenically unsaturated monomers that can be used, mention may be made of trifluoroethylene, tetrafluoroethylene and hexafluoropropylene and also ethylenically unsaturated monomers containing both at least one fluorine atom and at least one chlorine atom, such as chlorotrifluoroethylene.

Particular preference is accorded to vinylidene fluoride polymers. The expression "vinylidene fluoride polymer" is understood to mean, for the purposes of the present invention, any polymer containing at least approximately 50% by weight of monomer units derived from vinylidene fluoride, therefore both the homopolymers of vinylidene fluoride, and the copolymers of vinylidene fluoride with one or more advantageously fluorinated, ethylenically unsaturated monomers. As examples of other fluorinated ethylenically unsaturated monomers that can be used, mention may be made of vinyl fluoride, trifluoroethylene, tetrafluoroethylene, hexafluoropropylene and also chlorotrifluoroethylene.

The expression "polymers derived from chlorinated ethylenically unsaturated monomers" is understood to mean the homopolymers of these monomers and the copolymers that these monomers form with at least one other halogenated ethylenically unsaturated monomer and/or one other unhalogenated ethylenically unsaturated monomer such as vinyl esters, acrylic or methacrylic monomers, styrene monomers and olefin monomers. These polymers comprise advantageously at least 50%, preferably at least 60%, particularly preferably at least 70% by weight of monomer units derived from a chlorinated ethylenically unsaturated monomer.

The expression "chlorinated ethylenically unsaturated monomers" is understood to mean the ethylenically unsaturated chlorinated monomers that are aliphatic and that have, as the only heteroatom(s), one or more chlorine atoms. As examples of chlorinated ethylenically unsaturated monomers for which the number of chlorine atoms is equal to 1, mention may be made of allyl chloride, crotyl chloride and vinyl chloride. As an example of a chlorinated ethylenically unsaturated monomer for which the number of chlorine atoms is equal to 2, mention may be made of vinylidene chloride.

The polymer derived from halogenated ethylenically unsaturated monomers is particularly preferably a chlorinated polymer.

Particular preference is accorded to vinyl chloride polymers. The expression "vinyl chloride polymer" is understood to mean, for the purposes of the present invention, any polymer containing at least approximately 50% by weight, preferably at least 60%, particularly preferably at least 70% by weight and more particularly preferably at least 85% by weight of monomer units derived from vinyl chloride, therefore both the homopolymers of vinyl chloride, and the copolymers of vinyl chloride with one or more ethylenically unsaturated monomers. As examples of ethylenically unsaturated monomers that are copolymerizable with vinyl chloride, mention may be made of fluorinated monomers such as vinylidene fluoride, vinyl esters such as vinyl acetate, acrylic monomers such as *n*-butyl acrylate, styrene monomers such as styrene, and olefin monomers such as ethylene, propylene and butadiene.

The process for preparing polymers according to the invention makes it possible to obtain excellent results when it is applied to the aqueous suspension homopolymerization of vinyl chloride.

The expression "aqueous suspension polymerization" is understood to mean polymerization using oil-soluble initiators, in this case in particular dialkyl peroxydicarbonates, in the presence of dispersants, such as for example, water-soluble cellulose ethers, partially saponified polyvinyl acetates (also known as polyvinyl alcohols) and mixtures thereof. It is also possible to use surfactants at the same time as the dispersants. The amount of dispersant used generally varies between 0.7 and 2.0‰ by weight relative to the monomer(s).

In the preferred case of the polymerization of vinyl chloride, it may also be carried out in aqueous microsuspension.

The expression "polymerization in aqueous microsuspension", also called polymerization in (partially or completely) homogenized aqueous dispersion, is understood to mean the polymerization, also in aqueous suspension, in which oil-soluble initiators, in this case in particular dialkyl peroxydicarbonates, are used, and an emulsion of monomer droplets is prepared by virtue of powerful mechanical stirring and the presence of emulsifiers, such as for example, alkali metal or ammonium carboxylates and alkyl sulphonates, optionally in combination with oil-soluble cosurfactants, such as long-chain alcohols, for example cetyl alcohol.

The expression "an initiator comprising a dialkyl peroxydicarbonate" is understood to mean, for the purposes of the present invention, that besides the dialkyl peroxydicarbonate, other standard initiators may be used at the same time in the polymerization process of the invention. As examples of such other initiators, mention may be made of *t*-butyl peroxyisopropylcarbonate, *t*-butyl peroxy-n-decanoate, *t*-butyl peroxyacetate, 1,1,3,3-tetramethylbutyl peroxy-methoxyacetate, *t*-butyl peroxypivalate, *t*-amyl peroxypivalate, di-*t*-butyl peroxide, 2-2-bis(2,2-dimethylpropanolperoxy)-4-methylpentane, diacyl peroxides, such as diacetyl peroxide; di-*n*-propionyl peroxide, diisobutyryl peroxide, dibenzoyl peroxide, diisobutanoyl peroxide, dioctanoyl peroxide, dilauroyl peroxide, dicumyl peroxide, di-*t*-amyl peroxide, *t*-butyl per-2-ethylhexanoate, *t*-butyl peroxymaleate, cumyl peroxyneodecanoate and *t*-amyl peroxyneodecanoate, cumene hydroperoxide, pinane hydroperoxide, *p*-menthane hydroperoxide; nitriles, such as 2,2'-azobis(methoxy-2,4-dimethylvaleronitrile), 2,2'-azobis(2,4-dimethylvaleronitrile), and similar compounds; azo compounds; dicycloalkyl peroxydicarbonates, such as dicyclohexyl peroxydicarbonate; and dialkyl peroxydicarbonates having long alkyl chains, such as diethylhexyl peroxydicarbonate.

However, it is preferred to initiate the polymerization with the exclusive use of at least one dialkyl peroxydicarbonate. In this preferred case, the initiator is constituted of at least one dialkyl peroxydicarbonate, with the preferences defined above for the dialkyl peroxydicarbonate. The expression "constituted of at least one dialkyl peroxydicarbonate" is understood to mean, for the purposes of the present invention, that the initiator is constituted of one or more dialkyl peroxydicarbonates. Particularly preferably, it is constituted of a single dialkyl peroxydicarbonate, with the preferences defined above.

The expression "dialkyl peroxydicarbonate" is used without distinction in the singular and in the plural below.

It is also understood that the dialkyl peroxydicarbonate is advantageously used in the polymerization at least partly in the form of an organic solution in a liquid organic solvent (S). The expression "at least partly" should be understood, according to the present description, as meaning that the dialkyl peroxydicarbonate may be used in the polymerization in the form of an organic solution in a liquid organic solvent (S) and, in addition, in any other form. Preferably, the dialkyl peroxydicarbonate is used in the polymerization in the form of an organic solution in a liquid organic solvent (S).

According to the variant for preparing the organic solution of a dialkyl peroxydicarbonate according to the invention, the dialkyl peroxydicarbonate may be used in the polymerization:
- either in the form of an organic solution comprising the dialkyl peroxydicarbonate, the liquid organic solvent (S) and water, in particular water resulting from the preparation of the organic solution, i.e. water which may especially contain water-soluble compounds resulting from this preparation, in particular resulting from preparation variants 1 and 1.1 described above;
- or in the form of an organic solution constituted essentially of the dialkyl peroxydicarbonate and of the liquid organic solvent (S), in particular resulting from preparation variant 2 described above.

The use, in the polymerization, of an organic solution constituted essentially of the dialkyl peroxydicarbonate and of the liquid organic solvent (S), in particular resulting from preparation variant 2, is preferred.

It is also understood that the dialkyl peroxydicarbonates used in the polymerization at least partly in the form of an organic solution, may be introduced, completely or partly, after the start of the polymerization (delayed). The delayed use of a portion of the dialkyl peroxydicarbonate is advantageous for improving the polymerization kinetics or else for producing resins with a low K-value (produced at high temperature) that exhibit good thermal stability.

The total amount of initiator used generally ranges from around 0.15 to 3‰ and more particularly still from around 0.20 to 1.5‰ by weight approximately relative to the monomer(s) used.

Apart from the distinctive feature of the use of a dialkyl peroxydicarbonate at least partly in the form of an organic solution in a liquid organic solvent (S) corresponding to the formula (I), the general conditions of the polymerization do not differ from those customarily used for the preparation, in aqueous suspension or in aqueous microsuspension, of polymers derived from halogenated ethylenically unsaturated monomers, particularly of polymers derived from ethylenically unsaturated monomers containing chlorine and more particularly of vinyl chloride polymers.

The polymerization temperature is customarily between around 40°C and 80°C.

At the end of the polymerization, the polymers produced according to the process of the invention are isolated in a conventional manner from their polymerization medium, generally after having been purified of residual monomer(s).

The organic solutions of a dialkyl peroxydicarbonate according to the invention or that are obtained by the preparation process according to the invention have many advantages. Indeed, given that they are prepared *"ex situ*", the productivity of the polymerization process using them is not affected and in addition they may give rise to a delayed feeding of the polymerization reactor, which is advantageous for improving the polymerization kinetics or else for producing resins with a low K-value (produced at high temperature) that exhibit good thermal stability. These solutions are also ready-to-use and do not necessarily require the prior elimination of the solvent. One and the same solution may also be used to feed several polymerization reactors. Next, the solutions obtained can be conveyed without danger and do not lead to problems of deposits in the pipes.

One crucial advantage of the organic dialkyl peroxydicarbonate solution according to the invention or that is obtained by the process according to the invention is that the compounds corresponding to formula (I) (solvents (S)) exhibit the property of being incorporated into the growing macromolecular chains of the polymers, derived from halogenated ethylenically unsaturated monomers, synthesized in the presence of the dialkyl peroxydicarbonate solution.

The expression "compounds (solvents (S)) exhibiting the property of being incorporated into the growing macromolecular chains of the polymers" is understood to mean, according to the present description, the solvents that are incorporated into said chains by any type of reaction. This incorporation of said solvents may, for example, be the result of transfer reactions or of copolymerization reactions. Without this hypothesis in any way limiting the scope of the invention, the incorporation of the solvents of formula (I) by a copolymerization reaction, via their carbon-carbon double bond, appears to be the most common reaction mechanism leading to the most effective incorporation.

Irrespective of the mechanism according to which the solvent (S) corresponding to the formula (I) is incorporated into the growing macromolecular chains of the polymers, this incorporation is advantageously such that the amount of solvent (S) corresponding to the formula (I), that can be detected in the polymer synthesized, represents advantageously less than 0.5% by weight, preferably less than 0.1% by weight, very particularly less than 0.05% by weight of the amount of this solvent present in the dialkyl peroxydicarbonate solution used. In particular, when, in the chemical structure of the solvents (S) corresponding to the formula (I), A (and optionally B) represent(s) a halogenated functional group (A1), these solvents are virtually undetectable in said polymers, giving the latter a high purity.

The preferred organic solutions constituted essentially of the dialkyl peroxydicarbonate and of the liquid organic solvent (S) exhibit the additional advantage that, given that most of the impurities that appear during the preparation are water-soluble and are eliminated with the aqueous phase, the solutions obtained are very pure; they may also be stored, generally at low temperature (below 5°C), without drawback for longer periods of time (several months) without notable loss of activity. Relatively large amounts of the organic dialkyl peroxydicarbonate solution, sufficient for a large number of polymerization cycles, may therefore be prepared and then stored to be used as required.

Should the disclosure of any patents, patent applications, and publications which are incorporated herein by reference conflict with the description of the present application to the extent that it may render a term unclear, the present description shall take precedence.

The following examples are intended to illustrate the invention without however limiting the scope thereof.

### Example 1A - Preparation of a diethyl peroxydicarbonate solution

Introduced into a stirred, 1 l reactor were 515 cm³ of demineralized water and 112 g of sodium chloride. Next, the following were successively introduced: 20.4 g of diethyl chloroformate and 2.98 g of hydrogen peroxide in the form of a solution containing 352 g/kg, and finally, very slowly (over 30 minutes), 7.22 g of sodium hydroxide in the form of a solution containing 200 g/l so as to keep the temperature below 15°C. 10 minutes after the end of the introduction of the sodium hydroxide solution, 30 g of allyl chloride (solvent (S)) were introduced. After having kept the reaction medium under stirring for 15 minutes, the stirring was stopped. The aqueous phase (dense phase) was then separated by settling, and the organic phase was recovered. The solution of diethyl peroxydicarbonate in allyl chloride thus manufactured was stored at 5°C for the purpose of the subsequent use thereof. Its diethyl peroxydicarbonate content (evaluated by analysis as detailed below) amounted to 394 g/kg.

The diethyl peroxydicarbonate content has been evaluated as follows. 0.6 g (w) of the diethyl peroxydicarbonate solution have been weighted and placed in a 200 ml conical flask before adding 10 ml of chloroform and dissolving sample. The flask has then been placed under a stream of nitrogen during 1 to 2 minutes with adjustment of the flow so that it caused a slight dimple on the surface of the liquid. The flow has been kept for the following additions. 2 ml of a saturated solution of potassium iodide in water has then been added. 15 ml of glacial acetic acid and 1 drop of a solution of iron (III) chloride in glacial acetic acid (prepared by dissolving 5 mg of FeCl₃.6H₂O in 5 ml of glacial acetic acid) have afterwards been added. The flask has then been covered, its content mixed and let react in darkness for 10 minutes. 50 ml of demineralized water have then been added. Titration with a sodium thiosulfate solution 0.1N was then performed with addition of 3 ml of a 1 % starch solution in water near to the end. Titration was performed until the blue colour disappeared. Blank test titration was performed simultaneously on the reagents only (no diethyl peroxydicarbonate solution).

The diethyl peroxydicarbonate content (in g/kg) has then been calculated with the following equation (a - b) * N * 0.5 * 178.142 / w where a is the volume in ml of sodium thiosulfate added for the titration of the diethyl peroxydicarbonate solution, b is the volume in ml of sodium thiosulfate added for the blank test titration, N is the normality of the solution of sodium thiosulfate and w is the weight of the solution in g.

### Example 1B - Preparation of a halogenated polymer using the dimethyl peroxydicarbonate solution prepared in Example 1A

Introduced, at room temperature and with stirring (150 rpm), into a reactor with a 3 l capacity equipped with a stirrer and a jacket, were 1.3 kg of demineralized water, 1.3 g of polyvinyl alcohol (degree of hydrolysis: 72 mol%) and 0.4 g of diethyl peroxydicarbonate in the form of the solution prepared in Example 1A. The reactor was sealed, the stirring was stopped, and the reactor was put under a partial vacuum (60 mmHg absolute), which was maintained for 5 minutes. Next, 1 kg of vinyl chloride was introduced and the stirring was restarted (550 rpm). The medium was heated at 61°C, after which cold water was circulated in the jacket. The moment when the polymerization medium reached 61°C was considered to be the start of the polymerization (time = to). When the pressure in the reactor had dropped by 1 kg/cm², the polymerization was stopped by successively performing: an introduction of 0.05 g of ammonia and cooling. The polyvinyl chloride (PVC) produced was isolated in a conventional manner from the aqueous suspension after degassing of the unconverted vinyl chloride. 0.8 kg of polyvinyl chloride was recovered. Example 1C - Preparation of a halogenated polymer using the diethyl peroxydicarbonate solution prepared in Example 1A diluted to 169 g/kg

Example 1A was reproduced except that the solution of diethyl peroxydicarbonate in allyl chloride was diluted by addition of allyl chloride so that the final concentration was 169 g/kg.

### Examples 2(R)A and 2(R)B

These examples are given by way of comparison.

### Example 2(R)A

Example 1A was reproduced except that the allyl chloride was replaced by dibutyl maleate. The content of diethyl peroxydicarbonate in the solution (evaluated by analysis as described above) amounted to 245 g/kg.

### Example 2(R)B

Example 1B was reproduced except that the diethyl peroxydicarbonate solution therein, prepared in Example 1A, was replaced with the solution prepared in Example 2(R)A.

### Examples 3A and 3B

### Example 3A

Example 1A was reproduced except that the allyl chloride was replaced by allyl acetate. The content of diethyl peroxydicarbonate in the solution (evaluated by analysis as described above) amounted to 369 g/kg.

### Example 3B

Example 1B was reproduced except that the diethyl peroxydicarbonate solution therein, prepared in Example 1A, was replaced with the solution prepared in Example 3A.

### Examples 4A and 4B

### Example 4A

Example 1A was reproduced except that the allyl chloride was replaced by 3-chloro-2-methyl-1-propene. The content of diethyl peroxydicarbonate in the solution (evaluated by analysis as described above) amounted to 293 g/kg.

### Example 4B

Example 1B was reproduced except that the diethyl peroxydicarbonate solution therein, prepared in Example 1A, was replaced with the solution prepared in Example 4A.

### Examples 5(R)A and 5(R)B

These examples are given by way of comparison.

### Example 5(R)A

Example 1A was reproduced except that the allyl chloride was replaced by 1-octene. The content of diethyl peroxydicarbonate in the solution (evaluated by analysis as described above) amounted to 169 g/kg.

### Example 5(R)B

Example 1B was reproduced except that the diethyl peroxydicarbonate solution therein, prepared in Example 1A, was replaced with the solution prepared in Example 5(R)A.

### Examples 6(R)A and 6(R)B

These examples are given by way of comparison.

### Example 6(R)A

Example 1A was reproduced except that the allyl chloride was replaced by diethylhexyl adipate. The content of diethyl peroxydicarbonate in the solution (evaluated by analysis as described above) amounted to 278 g/kg.

### Example 6(R)B

Example 1 B was reproduced except that the diethyl peroxydicarbonate solution therein, prepared in Example 1A, was replaced with the solution prepared in Example 6(R)A.

### Example 7A

Example 1A was reproduced except that the allyl chloride was replaced by 1,3-dichloropropene (mixture of the cis stereoisomer (47%) and of the trans stereoisomer (53%)) and that the introduction, at the start, of sodium chloride was skipped. In this case, the organic phase was denser than the aqueous phase and was recovered first. The content of diethyl peroxydicarbonate in the solution (evaluated by analysis as described above) amounted to 289 g/kg.

### Example 7B

Example 1B was reproduced except that the diethyl peroxydicarbonate solution therein, prepared in Example 1A, was replaced with the solution prepared in Example 7A.

The table below collates the results obtained by the tests of Examples 1 to 7.

**Table**

| Examples | Nature of the solvent | Amount of solvent used (expressed as g per kg of PVC obtained) | Content of solvent in the PVC obtained | |
|---|---|---|---|---|
| | | | as g per kg of PVC obtained | as % of the amount of solvent used |
| 1A/1B | allyl chloride | 0.8 | < 0.02 × 10⁻³ | < 2.5 × 10⁻³ |
| 1A/1C | allyl chloride | 2.5 | < 0.02 × 10⁻³ | < 2.5 × 10⁻³ |
| 2(R)A/2(R)B | dibutyl maleate | 1.5 | 0.2 | 13 |
| 3A/3 B | allyl acetate | 0.9 | 0.28 × 10⁻³ | 0.03 |
| 4A/4B | 3-chloro-2-methyl-1-propene | 1.2 | 0.12 × 10⁻³ | 0.01 |
| 5(R)A/5(R)B | 1-octene | 2.5 | 0.06 | 2.7 |
| 6(R)A/6(R)B | diethylhexyl adipate | 1.3 | 1.2 | 92 |
| 7A/7B | 1,3-dichloropropene | 1.6 | 0.2 × 10⁻³ | 0.01 |

### Methods for determining the content of solvent (S) in the PVC obtained Allyl chloride

The allyl chloride in the PVC was assayed by gas chromatography (GC) after dissolving the PVC in dimethylacetamide.

GC of the headspace (gas phase on top of the solution) surmounting the PVC was carried out, by external calibration with a flame ionization detector (FID). The calibration was established by measured additions of PVC.

500 mg of sample were placed in a vial of around 20 ml and thermostatically controlled at 90°C for 120 minutes.

The GC was carried out using a CP-Sil 5 CB column, characterized by a length of 50 m, an internal diameter of 0.32 mm and a film thickness of 1.2 µm. The temperatures applied were 35°C (5 min) - 6°C/min - 100°C (5 min)-25°C/min - 260°C (5 min) for the oven programming, 150°C for the injector and 280°C for the detector. The injection was of split type and the amount injected was 30 ml/min. The carrier gas was helium, the flow rate of which was 2.5 ml/min. The auxiliairy gases were air (300 ml/min), hydrogen (30 ml/min) and a helium make-up of 27.5 ml/min.

### Dibutyl maleate

The dibutyl maleate in the PVC was assayed by GC after dissolving the PVC in dimethylacetamide.

Added to 5 ml of the solution of the sample were 100 µl of a solution of diisobutyl phthalate (at 1 g/kg) in chloroform.

The samples were subjected to successive extractions: 2.5 g of sample were extracted successively in a sealed flask using *t*-butyl methyl ether at 85°C. After 4 to 5 successive extractions, the extracts were brought together and concentrated to 5 ml and then made up to a volume of 10 ml.

The extracts were then analysed by GC carried out using an Ultra 2 column, characterized by a length of 25 m, an internal diameter of 0.2 mm and a film thickness of 0.11 µm. The temperatures applied were 100°C (1 min) - 15°C/min-300°C for the oven programming, 280°C for the injector and 290°C for the detector. The injection was of split type and the amount injected was 80 ml/min. The carrier gas was helium, the flow rate of which was 1 ml/min. The auxiliairy gases were air (300 ml/min), hydrogen (30 ml/min) and a helium make-up of 29 ml/min.

### Allyl acetate

The allyl acetate in the PVC was assayed by GC under the same conditions as the assaying of the allyl chloride.

### 3 -chloro-2-methyl-1-propene

The 3-chloro-2-methyl-1-propene in the PVC was assayed by GC under the same conditions as the allyl chloride assay except that the sample was thermostatically controlled at 90°C for 135 minutes.

### 1-Octene

The 1-octene in the PVC was assayed by GC after dissolving the PVC in dimethylacetamide.

Added to 5 ml of the solution of the sample were 50 µl of a solution of *n-*nonane (at 10 g/kg) in *t*-butyl methyl ether.

The samples were subjected to successive extractions under the same conditions as for the dibutyl maleate.

The extracts were then analysed by GC carried out using a CP-Sil 5 CB column. The temperatures applied were 35°C (5 min) - 6°C/min - 120°C (25 min) - 25°C/min - 280°C (2 min) for the oven programming, 150°C for the injector and 280°C for the detector. The injection was of split type and the amount injected was 30 ml/min. The carrier gas was helium, the flow rate of which was 2.5 ml/min. The auxiliairy gases were air (300 ml/min), hydrogen (30 ml/min) and a helium make-up of 27.5 ml/min.

### Diethylhexyl adipate

The diethylhexyl adipate in the PVC was assayed by GC under the same conditions as the dibutyl maleate assay.

### 1,3-dichloropropene

The 1,3-dichloropropene in the PVC was assayed by GC under the same conditions as the allyl chloride assay except that the sample was thermostatically controlled at 90°C for 135 minutes.

It was observed that, in the PVC obtained, less than 1% of the amount employed of the solvents (S) used for preparing the initiators according to the invention (Examples 1, 3, 4 and 7) remained. The content of allyl chloride was even below the detection threshold (Example 1). On the other hand, 13% of dibutyl maleate (Example 2(R)) and 2.7% of octene (Example 5(R)), of the amount employed, remained despite the presence in their molecule of an ethylenic unsaturation which, according to the prior art (see documents (D1) and (D2)) is reputed to favour their incorporation. As regards the diethylhexyl adipate (Example 6(R)), 92% of the amount of this solvent employed remained in the PVC obtained.

## Claims

1. Process for preparing an organic solution of a dialkyl peroxydicarbonate in a liquid organic solvent (S), carried out at least partly in the presence of the liquid organic solvent (S) and comprising at least one step during which, in a medium containing water, an alkyl haloformate is reacted with an inorganic peroxide, the liquid organic solvent (S) being chosen from the compounds corresponding to the empirical formula:
A - (CₙH(₂ₙ₋₂₎) - B (I)
in which:
- A represents a halogen functional group (A1) or a functional group (A2) corresponding to the structure R-C(=O)-O- in which R is chosen from hydrogen; chlorine; substituted or unsubstituted, linear or branched C₁-C₂₀ alkyl radicals; substituted or unsubstituted C₃-C₂₂ alkenyl radicals; substituted or unsubstituted C₆-C₁₀ cycloalkyl radicals; substituted or unsubstituted C₇-C₁₂ aralkyl radicals, and substituted or unsubstituted C₆-C₁₀ aryl radicals;
- B represents either a hydrogen atom or, when A is not (A2), a second halogen functional group (A1); and
- n is such that 3 ≤ n ≤ 8.

2. Preparation process according to Claim 1, **characterized in that** the dialkyl peroxydicarbonate is diethyl peroxydicarbonate.

3. Preparation process according to Claim 1 or 2, **characterized in that**, in formula (I), the halogen functional group (A1) is chlorine.

4. Preparation process according to any one of Claims 1 to 3, **characterized in that** the solvent (S) is chosen from the compounds corresponding to the formula (I) in which A represents a halogen functional group (A1) and B a hydrogen atom.

5. Preparation process according to Claim 4, **characterized in that** the solvent (S) is chosen from the C₃ - C₈ chloro-1-alkenes in which the alkenyl radical is linear or branched.

6. Preparation process according to Claim 5, **characterized in that** the solvent (S) is chosen from 3-chloro-1-propene and 3-chloro-2-methyl-1-propene.

7. Preparation process according to any one of Claims 1 to 3, **characterized in that** the solvent (S) is chosen from the compounds corresponding to the formula (I) in which A represents a functional group (A2) and B a hydrogen atom.

8. Preparation process according to Claim 7, **characterized in that** the solvent (S) is chosen from allyl formate, acetate, propionate and butyrate.

9. Preparation process according to any one of Claims 1 to 3, **characterized in that** the solvent (S) is chosen from the compounds corresponding to the formula (I) in which A and B each represent a halogen functional group (A1).

10. Preparation process according to Claim 9, **characterized in that** the solvent (S) is chosen from the stereoisomers of 1,3-dichloropropene and mixtures thereof.

11. Preparation process according to any one of Claims 1 to 10, **characterized in that** the dialkyl peroxydicarbonate is prepared, in a first step, by reacting, in a medium containing water and optionally the solvent (S), the alkyl haloformate with the inorganic peroxide and **in that**, in a second step, the dialkyl peroxydicarbonate prepared during the first step is separated.

12. Organic solution of a dialkyl peroxydicarbonate in a liquid organic solvent (S) chosen from the compounds corresponding to the empirical formula:
A - (CₙH(₂ₙ₋₂₎) - B (I)
in which:
- A represents a halogen functional group (A1) or a functional group (A2) corresponding to the structure R-C(=O)-O- in which R is chosen from hydrogen; chlorine; substituted or unsubstituted, linear or branched C₁-C₂₀ alkyl radicals; substituted or unsubstituted C₃-C₂₂ alkenyl radicals; substituted or unsubstituted C₆-C₁₀ cycloalkyl radicals; substituted or unsubstituted C₇-C₁₂ aralkyl radicals, and substituted or unsubstituted C₆-C₁₀ aryl radicals;
- B represents either a hydrogen atom or, when A is not (A2), a second halogen functional group (A1); and
- n is such that 3 ≤ n ≤ 8.

13. Organic solution according to Claim 12, **characterized in that** it is essentially constituted of the dialkyl peroxydicarbonate and the liquid organic solvent (S).

14. Process for preparing a polymer derived from one or more halogenated ethylenically unsaturated monomers, comprising a polymerization step during which at least one halogenated ethylenically unsaturated monomer is polymerized in aqueous suspension or in aqueous microsuspension in the presence of an initiator comprising at least one dialkyl peroxydicarbonate, used in the polymerization at least partly in the form of an organic solution in a liquid organic solvent (S) chosen from the compounds corresponding to the empirical formula:
A - (CₙH(₂ₙ₋₂₎) - B (I)
in which:
- A represents a halogen functional group (A1) or a functional group (A2) corresponding to the structure R-C(=O)-O- in which R is chosen from hydrogen; chlorine; substituted or unsubstituted, linear or branched C₁-C₂₀ alkyl radicals; substituted or unsubstituted C₃-C₂₂ alkenyl radicals; substituted or unsubstituted C₆-C₁₀ cycloalkyl radicals; substituted or unsubstituted C₇-C₁₂ aralkyl radicals, and substituted or unsubstituted C₆-C₁₀ aryl radicals;
- B represents either a hydrogen atom or, when A is not (A2), a second halogen functional group (A1); and
- n is such that 3 ≤ n ≤ 8.

15. Process for preparing a polymer according to Claim 14, applied to the homopolymerization, in aqueous suspension, of vinyl chloride.

## Patentansprüche

1. Verfahren zur Herstellung einer organischen Lösung eines Dialkylperoxydicarbonats in einem flüssigen organischen Lösungsmittel (S), das zumindest teilweise in Gegenwart des flüssigen organischen Lösungsmittels (S) durchgeführt wird und mindestens einen Schritt umfasst, bei dem ein Halogenameisensäurealkylester in einem Wasser enthalten den Medium mit einem anorganischen Peroxid umgesetzt wird, wobei das flüssige organische Lösungsmittel (S) aus den Verbindungen mit der Summenformel:
A - (CₙH₍₂ₙ₋₂₎) - B (I)
in der:
- A für eine halogenfunktionelle Gruppe (A1) oder eine funktionelle Gruppe (A2) mit der Struktur R-C(=O)-O-, worin R aus Wasserstoff; Chlor; substituierten oder unsubstituierten, linearen oder verzweigten C₁-C₂₀-Alkylresten; substituierten oder unsubstituierten C₃-C₂₂-Alkenyl resten; substituierten oder unsubstituierten C₆-C₁₀-Cycloalkylresten; substituierten oder un substituierten C₇-C₁₂-Aralkylresten und substituierten oder unsubstituierten C₆-C₁₀-Arylresten ausgewählt ist, steht;
- B entweder für ein Wasserstoffatom oder dann, wenn A nicht für (A2) steht, eine zweite halogen funktionelle Gruppe (A1) steht; und
- n so beschaffen ist, dass 3 ≤ n ≤ 8;
ausgewählt wird.

2. Herstellungsverfahren nach Anspruch 1, **dadurch gekennzeichnet, dass** es sich bei dem Dialkylperoxydicarbonat um Diethylperoxydicarbonat handelt.

3. Herstellungsverfahren nach Anspruch 1 oder 2, **dadurch gekennzeichnet, dass** in Formel (I) die halogenfunktionelle Gruppe (A1) Chlor ist.

4. Herstellungsverfahren nach einem der Ansprüche 1 bis 3, **dadurch gekennzeichnet, dass** das Lösungs mittel (S) aus den Verbindungen mit der Formel (I), in der A für eine halogenfunktionelle Gruppe (A1) steht und B für ein Wasserstoffatom steht, ausgewählt wird.

5. Herstellungsverfahren nach Anspruch 4, **dadurch gekennzeichnet, dass** das Lösungs mittel (S) aus den C₃-C₈-Chlor-1-alkenen, in denen der Alkenylrest linear oder verzweigt ist, ausgewählt wird.

6. Herstellungsverfahren nach Anspruch 5, **dadurch gekennzeichnet, dass** das Lösungs mittel (S) aus 3-Chlor-1-propen und 3-Chlor-2-methyl-1-propen ausgewählt wird.

7. Herstellungsverfahren nach einem der Ansprüche 1 bis 3, **dadurch gekennzeichnet, dass** das Lösungs mittel (S) aus den Verbindungen mit der Formel (I), in der A für eine funktionelle Gruppe (A2) steht und B für ein Wasserstoffatom steht, ausgewählt wird.

8. Herstellungsverfahren nach Anspruch 7, **dadurch gekennzeichnet, dass** das Lösungs mittel (S) aus Allylformiat, -acetat, -propionat und -butyrat ausgewählt wird.

9. Herstellungsverfahren nach einem der Ansprüche 1 bis 3, **dadurch gekennzeichnet, dass** das Lösungsmittel (S) aus den Verbindungen mit der Formel (I), in der A und B jeweils für eine halogenfunktionelle Gruppe (A1) stehen, ausgewählt wird.

10. Herstellungsverfahren nach Anspruch 9, **dadurch gekennzeichnet, dass** das Lösungs mittel (S) aus den Stereoisomeren von 1,3-Dichlorpropen und Gemischen davon ausgewählt wird.

11. Herstellungsverfahren nach einem der Ansprüche 1 bis 10, **dadurch gekennzeichnet, dass** das Dialkylperoxydicarbonat dadurch hergestellt wird, dass man in einem ersten Schritt den Halogenameisensäurealkylester in einem Wasser und gegebenenfalls das Lösungsmittel (S) enthaltenden Medium mit dem anorganischen Peroxid umsetzt und in einem zweiten Schritt das im ersten Schritt hergestellte Dialkylperoxydicarbonat abtrennt.

12. Organische Lösung eines Dialkylperoxydicarbonats in einem flüssigen organischen Lösungsmittel (S), das aus den Verbindungen mit der Summenformel:
A - (CₙH₍₂ₙ₋₂₎) - B (I)
in der:
- A für eine halogenfunktionelle Gruppe (A1) oder eine funktionelle Gruppe (A2) mit der Struktur R-C(=O)-O-, worin R aus Wasserstoff; Chlor; substituierten oder unsubstituierten, linearen oder verzweigten C₁-C₂₀-Alkylresten; substituierten oder unsubstituierten C₃-C₂₂-Alkenylresten; substituierten oder unsubstituierten C₆-C₁₀-Cycloalkylresten; substituierten oder un substituierten C₇-C₁₂-Aralkylresten und substituierten oder unsubstituierten C₆-C₁₀-Arylresten ausgewählt ist, steht;
- B entweder für ein Wasserstoffatom oder dann, wenn A nicht für (A2) steht, eine zweite halogen funktionelle Gruppe (A1) steht; und
- n so beschaffen ist, dass 3 ≤ n ≤ 8;
ausgewählt ist.

13. Organische Lösung nach Anspruch 12, **dadurch gekennzeichnet, dass** sie im Wesentlichen aus dem Dialkylperoxydicarbonat und dem flüssigen organischen Lösungsmittel (S) besteht.

14. Verfahren zur Herstellung eines Polymers, das sich von einem oder mehreren halogenierten ethylenisch ungesättigten Monomeren ableitet, umfassend einen Polymerisationsschritt, bei dem man mindestens ein halogeniertes ethylenisch ungesättigtes Monomer in einer wässrigen Suspension oder in einer wässrigen Mikrosuspension in Gegenwart eines Initiators, der mindestens ein Dialkylperoxydicarbonat, das bei der Polymerisation zumindest teilweise in Form einer organischen Lösung in einem flüssifen organischen Lösungsmittel (S), das aus den Verbindungen mit der Summenformel:
A - (CₙH₍₂ₙ₋₂₎) - B (I)
in der:
- A für eine halogenfunktionelle Gruppe (A1) oder eine funktionelle Gruppe (A2) mit der Struktur R-C(=O)-O-, worin R aus Wasserstoff; Chlor; substituierten oder unsubstituierten, linearen oder verzweigten C₁-C₂₀-Alkylresten; substituierten oder unsubstituierten C₃-C₂₂-Alkenylresten; substituier ten oder unsubstituierten C₆-C₁₀-Cycloalkyl resten; substituierten oder un substituierten C₇-C₁₂-Aralkylresten und substituier ten oder unsubstituierten C₆-C₁₀-Arylresten ausgewählt ist, steht;
- B entweder für ein Wasserstoffatom oder dann, wenn A nicht für (A2) steht, eine zweite halogenfunktionelle Gruppe (A1) steht; und
- n so beschaffen ist, dass 3 ≤ n ≤ 8;
ausgewählt wird, verwendet wird, umfasst, polymerisiert.

15. Verfahren zur Herstellung eines Polymers nach Anspruch 14, angewendet auf die Homopolymerisation von Vinylchlorid in wässriger Suspension.

## Revendications

1. Procédé de préparation d'une solution organique d'un peroxydicarbonate de dialkyle dans un solvant organique liquide (S), réalisé au moins partiellement en présence du solvant organique liquide (S) et comprenant au moins une étape durant laquelle, dans un milieu contenant de l'eau, on fait réagir un halogénoformate d'alkyle avec un peroxyde inorganique, le solvant organique liquide (S) étant choisi parmi les composés correspondant à la formule empirique:
A-(CₙH₍₂ₙ₋₂₎)-B (I)
dans laquelle :
- A représente un groupe fonctionnel halogène (A1) ou un groupe fonctionnel (A2) correspondant à la structure R-C(=O)-O- dans laquelle R est choisi parmi l'hydrogène; le chlore; les radicaux alkyles en C₁-C₂₀ substitués ou non substitués, linéaires ou ramifiés ; les radicaux alcényles en C₃-C₂₂ substitués ou non substitués; les radicaux cycloalkyles en C₆-C₁₀ substitués ou non substitués ; les radicaux aralkyles en C₇-C₁₂ substitués ou non substitués; et les radicaux aryles en C₆-C₁₀ substitués ou non substitués;
- B représente un atome d'hydrogène ou, quand A n'est pas (A2), un deuxième groupe fonctionnel halogène (A1); et
- n est tel que 3 ≤ n ≤ 8.

2. Procédé de préparation selon la revendication 1, **caractérisé en ce que** le peroxydicarbonate de dialkyle est le peroxydicarbonate de diéthyle.

3. Procédé de préparation selon la revendication 1 ou 2, **caractérisé en ce que**, dans la formule (I), le groupe fonctionnel halogène (A1) est le chlore.

4. Procédé de préparation selon l'une quelconque des revendications 1 à 3, **caractérisé en ce que** le solvant (S) est choisi parmi les composés correspondant à la formule (I) dans laquelle A représente un groupe fonctionnel halogène (A1) et B un atome d'hydrogène.

5. Procédé de préparation selon la revendication 4, **caractérisé en ce que** le solvant (S) est choisi parmi les chloro-1-alcènes en C₃-C₈ dans lesquels le radical alcényle est linéaire ou ramifié.

6. Procédé de préparation selon la revendication 5, **caractérisé en ce que** le solvant (S) est choisi parmi le 3-chloro-1-propène et le 3-chloro-2-méthyl-1-propène.

7. Procédé de préparation selon l'une quelconque des revendications 1 à 3, **caractérisé en ce que** le solvant (S) est choisi parmi les composés correspondant à la formule (I) dans laquelle A représente un groupe fonctionnel (A2) et B un atome d'hydrogène.

8. Procédé de préparation selon la revendication 7, **caractérisé en ce que** le solvant (S) est choisi parmi le formiate, l'acétate, le propionate et le butyrate d'allyle.

9. Procédé de préparation selon l'une quelconque des revendications 1 à 3, **caractérisé en ce que** le solvant (S) est choisi parmi les composés correspondant à la formule (I) dans laquelle A et B représentent chacun un groupe fonctionnel halogène (A1).

10. Procédé de préparation selon la revendication 9, **caractérisé en ce que** le solvant (S) est choisi parmi les stéréoisomères de 1,3-dichloropropène et leurs mélanges.

11. Procédé de préparation selon l'une quelconque des revendications 1 à 10, **caractérisé en ce que** le peroxydicarbonate de dialkyle est préparé, dans une première étape, en faisant réagir, dans un milieu contenant de l'eau et optionnellement le solvant (S), l'halogénoformiate d'alkyle avec le peroxyde inorganique et **en ce que**, dans une deuxième étape, le peroxydicarbonate de dialkyle préparé durant la première étape est séparé.

12. Solution organique d'un peroxydicarbonate de dialkyle dans un solvant organique liquide (S) choisi parmi les composés correspondant à la formule empirique :
A-(CₙH₍₂ₙ₋₁₎)-B (I)
dans laquelle :
- A représente un groupe fonctionnel halogène (A1) ou un groupe fonctionnel (A2) correspondant à la structure R-C(=O)-O- dans laquelle R est choisi parmi l'hydrogène; le chlore; les radicaux alkyles en C₁-C₁₀ substitués ou non substitués, linéaires ou ramifiés ; les radicaux alcényle en C₃-C₂₂ substitués ou non substitués; les radicaux cycloalkyles en C₆-C₁₀ substitués ou non substitués ; les radicaux aralkyles en C₇-C₁₂ substitués ou non substitués; et les radicaux aryles en C₆-C₁₀ substitués ou non substitués;
- B représente un atome d'hydrogène ou, quand A n'est pas (A2), un deuxième groupe fonctionnel halogène (A1); et
- n est tel que 3 ≤ n ≤ 8.

13. Solution organique selon la revendication 12, **caractérisée en ce qu'**elle est essentiellement constituée du peroxydicarbonate de dialkyle et du solvant organique liquide (S).

14. Procédé de préparation d'un polymère dérivé d'un ou plusieurs monomères halogénés éthyléniquement insaturés, comprenant une étape de polymérisation durant laquelle au moins un monomère halogène éthyléniquement insaturé est polymérisé en suspension aqueuse ou en microsuspension aqueuse en présence d'un initiateur comprenant au moins un peroxydicarbonate de dialkyle, utilisé dans la polymérisation au moins partiellement sous la forme d'une solution organique dans un solvant organique liquide (S) choisi parmi les composés correspondant à la formule empirique:
A-(CₙH₍₂ₙ₋₂₎)-B (I)
dans laquelle :
- A représente un groupe fonctionnel halogène (A1) ou un groupe fonctionnel (A2) correspondant à la structure R-C(=O)-O- dans laquelle R est choisi parmi l'hydrogène; le chlore; les radicaux alkyles en C₁-C₂₀ substitués ou non substitués, linéaires ou ramifiés; les radicaux alcényles en C₃-C₂₂ substitués ou non substitués; les radicaux cycloalkyles en C₆-C₁₀ substitués ou non substitués; les radicaux aralkyles en C₇-C₁₂ substitués ou non substitués; et les radicaux aryles en C₆-C₁₀ substitués ou non substitués;
- B représente un atome d'hydrogène ou, quand A n'est pas (A2), un deuxième groupe fonctionnel halogène (A1); et
- n est tel que 3 ≤ n ≤ 8.

15. Procédé de préparation d'un polymère selon la revendication 14, appliqué à l'homopolymérisation, en suspension aqueuse, de chlorure de vinyle.
